(19) Europäisches Patentamt
European Patent Office
Office européen des brevets

(11) Veröffentlichungsnummer : **0 442 339 B1**

(12) **EUROPÄISCHE PATENTSCHRIFT**

(45) Veröffentlichungstag der Patentschrift :
**15.06.94 Patentblatt 94/24**

(51) Int. Cl.$^5$ : **C07D 213/30,** A01N 43/40

(21) Anmeldenummer : **91101436.3**

(22) Anmeldetag : **04.02.91**

# (54) Neue 3-substituierte Pyridinmethanole und diese enthaltende Fungizide.

(30) Priorität : **13.02.90 DE 4004317**

(43) Veröffentlichungstag der Anmeldung :
**21.08.91 Patentblatt 91/34**

(45) Bekanntmachung des Hinweises auf die Patenterteilung :
**15.06.94 Patentblatt 94/24**

(84) Benannte Vertragsstaaten :
**AT BE CH DE DK ES FR GB GR IT LI NL SE**

(56) Entgegenhaltungen :
**EP-A- 0 069 330**
**EP-A- 0 359 077**
**GB-A- 1 175 693**
**GB-A- 1 595 261**
**GB-A- 2 219 793**

(73) Patentinhaber : **BASF Aktiengesellschaft**
**Carl-Bosch-Strasse 38**
**D-67063 Ludwigshafen (DE)**

(72) Erfinder : **Zierke, Thomas, Dr.**
**Bahnhofstrasse 10**
**W-6737 Boehl-Iggelheim (DE)**
Erfinder : **Kuekenhoehner, Thomas, Dr.**
**Seidelstrasse 2**
**W-6710 Frankenthal (DE)**
Erfinder : **Ammermann, Eberhard, Dr.**
**Sachsenstrasse 3**
**W-6700 Ludwigshafen (DE)**
Erfinder : **Lorenz, Gisela, Dr.**
**Erlenweg 13**
**W-6730 Neustadt (DE)**



Anmerkung : Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Jouve, 18, rue Saint-Denis, 75001 PARIS

## Beschreibung

Die vorliegende Erfindung betrifft neue 3-Pyridinmethanole, fungizide Mittel, die die neuen Wirkstoffe enthalten, sowie Verfahren zur Bekämpfung von Pilzen mit diesen Wirkstoffen.

Es ist bekannt, 3-Pyridinmethanole als Fungizide zu verwenden. Aus US-A-3 396 224 ist beispielsweise alpha-(4-Chlorphenyl)-alpha-cyclopropyl-3-pyridinmethanol als fungizide Verbindung bekannt. Die fungizide Wirkung dieser Verbindung ist jedoch insbesondere bei kleinen Aufwandmengen häufig nicht ausreichend. Daraus ergibt sich die Aufgabe, neue 3-Pyridinmethanole mit anderer chemischer Struktur zu finden, die den bekannten 3-Pyridinmethanolen in ihrer fungiziden Wirkung überlegen sind.

Es wurde nun gefunden, daß 3-Pyridinmethanole der Formel 1

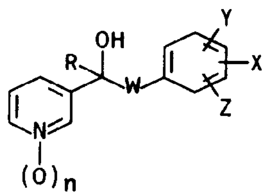

1,

in der R

für gegebenenfalls $C_1$-$C_4$-alkylsubstituiertes $C_3$-$C_8$-Cycloalkyl oder für gegebenenfalls $C_1$-$C_4$-alkylsubstituiertes $C_3$-$C_8$-Cycloalkenyl steht,

X, Y, Z

unabhängig voneinander Wasserstoff, Halogen, $C_1$-$C_4$-Alkyl, $C_1$-$C_4$-Alkoxy, $C_1$-$C_4$-Alkoximino, Halogen-$C_1$-$C_4$-alkyl, Cyano, Nitro oder gegebenenfalls durch Halogen substituiertes Phenyl oder gegebenenfalls durch Halogen substituiertes Phenoxy bedeuten,

W

für eine der Gruppen $-CH_2-$ oder $-CH_2CH_2-$ steht und

n

0 oder 1 bedeutet, sowie deren pflanzenverträgliche Säureadditionssalze eine überraschend bessere fungizide Wirkung als die aus US-3 396 224 bekannten 3-Pyridinmethanole haben.

R bedeutet beispielsweise Cyclopropyl, Cyclobutyl, Cyclopentyl, Cyclohexyl, Cycloheptyl, Cyclooctyl, 1-Methylcyclopropyl, 1-Ethylcyclopropyl, 1-Methylcyclopentyl, 4-Methylcyclohexyl, 4-Isopropylcyclohexyl, 4-tert.-Butylcyclohexyl, 2-Methylcyclopentyl, 2-Methylcyclohexyl.

Durch die Substituenten X, Y und Z und den Phenylring werden bis zu dreifach substituierte Phenylringe von der Erfindung umfaßt, wie z.B. Phenyl, Halogenphenyl, 4-Chlorphenyl, 4-Fluorphenyl, 4-Bromphenyl, 2-Chlorphenyl, 2-Fluorphenyl, 2-Bromphenyl, 2,4-Dichlorphenyl, 2-Brom-4-chlorphenyl, 2-Chlor-4-bromphenyl, 2-Chlor-4-fluorphenyl, 2,3-Dichlorphenyl, 2-Chlor-3-fluorphenyl, 2-Fluor-4-chlorphenyl, 2-Chlor-3-fluor-4-chlorphenyl, 2-Brom-4-fluorphenyl, 2-Brom-3-fluor-4-chlorphenyl, 2,6-Dichlorphenyl, 2-Trifluormethylphenyl, 4-Trifluormethylphenyl, $C_1$-$C_4$-Alkylphenyl, 2-Methylphenyl, 4-Methylphenyl, 4-tert.-Butylphenyl, 2-Chlor-4-methylphenyl, 2-Methyl-4-chlorphenyl, 2,4-Dimethylphenyl, 2,3-Dimethylphenyl, $C_1$-$C_4$-Alkoxyphenyl, 2-Methoxyphenyl, 4-Methoxyphenyl, 2-Chlor-4-methoxyphenyl, 2-Methyl-4-methoxyphenyl, 4-tert.-Butoxyphenyl, $C_1$-$C_4$-Alkoximinophenyl, 4-Methoximinophenyl, 4-Ethoximinophenyl, 2-Chlor-4-methoximinophenyl, 4-Cyanophenyl, 2-Chlor-4-cyanophenyl, 2-Cyano-4-chlorphenyl, 2-Cyanophenyl, 2-Nitrophenyl, 4-Nitrophenyl, 2-Chlor-4-nitrophenyl, Halogenphenoxyphenyl, 4-Phenoxyphenyl, 4-(4′-Chlorphenoxy)phenyl.

Für Pflanzen verträgliche Salze sind z.B. Säureadditionssalze mit anorganischen Mineralsäuren wie Hydrochloride, Hydrobromide, Sulfate, Phosphate, Nitrate, Salze mit Ameisensäure oder mit Alkylcarbonsäuren wie Acetate, 2-Ethylhexanoate, Oxalate, Salze mit Arylsulfonsäuren wie Benzolsulfonate, Toluolsulfonate, Dodecylbenzolsulfonate.

Die neuen Verbindungen der Formel 1 enthalten im allgemeinen chirale C-Atome. Sie werden im allgemeinen als Racemate oder ggf. als Diastereomerengemische erhalten. Diastereomerenreine Verbindungen lassen sich bei einigen der neuen Verbindungen durch Destillation, Säulenchromatographie oder aufgrund von Löslichkeitsunterschieden isolieren. Enantiomerenreine Verbindungen lassen sich z.B. durch Racematspaltung mit chiralen Hilfsreagenzien nach bekannten Methoden bzw. über diastereomere Salze erhalten. Für die Anwendung der neuen Verbindungen als Fungizide sind sowohl die Diastereomeren bzw. die Enantiomeren als auch die bei der Synthese anfallenden Stereoisomerengemische geeignet. Sie alle werden von der Erfindung umfaßt.

Verbindungen der Formel 1, in denen n = 1 bedeutet, werden aus den entsprechenden Verbindungen der Formel 1 mit n = 0 durch Behandlung mit einer Persäure oder mit Gemischen aus Säuren, wie Essigsäure, und Wasserstoffperoxid erhalten.

Die Herstellungsverfahren für Verbindungen der Formel 1, in denen n = 0 ist, lassen sich durch folgende Reaktionsgleichungen veranschaulichen:

Gl. 1:

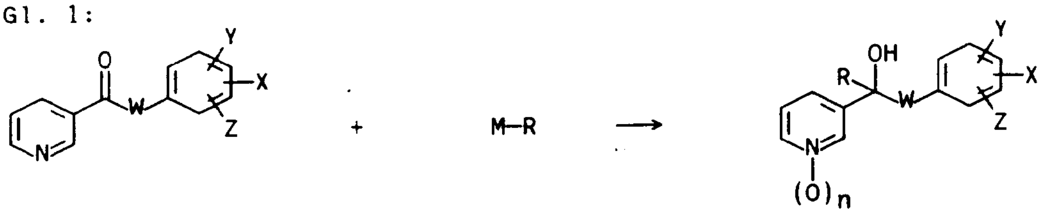

2 3 1

Gl. 2:

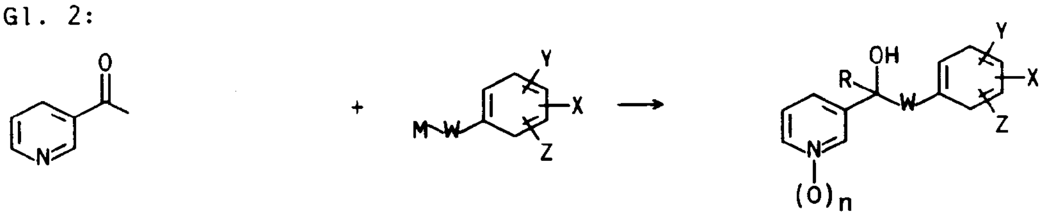

4 5 1

In der Formel 2 bis 5 haben W,X,Y,Z und R die gleiche Bedeutung wie in Formel 1. M kann in den Formeln 3 und 5 z.B. für Lithium, Natrium, Kalium oder die Reste MgCl oder MgBr stehen. Reaktionen mit metallorganischen Agenzien, wie durch Gl. 1 und 2 veranschaulicht, sind allgemein bekannte Reaktionen der organischen Chemie (siehe z.B. Organikum, 15. Aufl. 1977, S. 623 ff). Zweckmäßig wird die Reaktion so durchgeführt, daß man 1 bis 2 Äquivalente der Organometallverbindung in einem inerten Lösungsmittel, vorzugsweise Diethylether, Methyl-tert.-butylether, Tetrahydrofuran oder einem Gemisch derselben, vorlegt und die Ketonkomponente bei -50°C bis +50°C zudosiert.

Ketone der Formel 2 und 4 sind allgemein bekannte Verbindungen, die sich durch eine Vielzahl von bekannten Methoden herstellen lassen. Eine bevorzugte Methode besteht z.B. in der Alkylierung von alpha-Morpholino-3-pyridylacetonitril mit Alkylierungsmitteln der Formeln 6 bzw. 7 und nachfolgender Freisetzung der Ketonfunktion (E. Leete et al, JOC 37, S. 4465, 1972) oder in der Addition von 3-Pyridyllithium (W.E. Parham et al JOC 42 1977, S. 257 f) an Aldehyde der Formeln 8 bzw. 9 und nachfolgender Oxidation der erzeugten Carbinole.

R—A

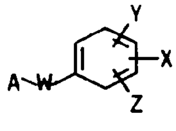

R—CHO

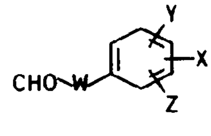

6 7 8 9

In den Formeln 6 bis 9 haben W,X,Y,Z und R die gleiche Bedeutung wie in Formel 1. A hat die Bedeutung einer nucleophil substituierbaren Gruppe wie z.B. Chlor, Brom, Jod, Tosylat, Benzolsulfonat oder Mesylat.

Die folgenden Beispiele beschreiben die Herstellung der neuen Verbindungen:

Beispiel 1

alpha-Cyclopropyl-alpha-(2,4-dichlorbenzyl)-3-pyridylcarbinol (Verbindung Nr. 1)

Zu einer Lösung von 2,4-Dichlorbenzylmagnesiumchlorid, 20 ml Diethylether ($Et_2O$), hergestellt aus 0,66 g (0,027 mol) Mg-Spänen und 5,3 g (0,027 mol) 2,4-Dichlorbenzylchlorid, werden 2 g (0,014 mol) 3-Pyridylcyclopropylketon, gelöst in 10 ml Tetrahydrofuran (THF) getropft. Nach 1 Stunde bei Raumtemperatur (20°C) wird nach Zugabe von gesättigter $NH_4$-Cl-Lsg. aufgearbeitet. Die wässrige Phase wird noch einmal mit Methylenchlorid ($CH_2Cl_2$) extrahiert. Die vereinigten organischen Phasen werden über $Na_2SO_4$ getrocknet, abfiltriert und eingeengt. Man erhält farblose Kristalle.
Ausbeute: 1.48 g (35 %), Schmelzpunkt: 98-100°C
$^1$H-NMR ($CDCl_3$/$TMS_{int}$): δ/ppm= 0,2-0,6 (m,4H), 1,35-1,5 (m,1H), 2,25 (s,breit,1H), 3,2 (d,1H), 3,4 (d,1H), 6,9-7,4 (m,4H), 7,7 (d,1H), 8,45 (d,1H), 8,7 (s,1H)

Beispiel 2

Herstellung von α-Cyclohexyl-α-(4-fluorphenethyl)-3-pyridylcarbinol (Verbindung Nr. 29)

Zu einer Lösung von Cyclohexylmagnesiumbromid in 20 ml Diethylether ($Et_2O$), hergestellt aus 0,7 g (0,03 mol) Mg-Spänen und 4,7 g (0,03 mol) Cyclohexylbromid, werden 6 g (0,026 mol) 1-(Pyrid-3-yl)-3-(4-fluorphenyl)-propanon, gelöst in 10 ml Tetrahydrofuran (THF), getropft. Nach 5stündiger Reaktionszeit bei Raumtemperatur (20°C) wird durch Zugabe von gesättigter $NH_4Cl$-Lösung aufgearbeitet. Die wäßrige Phase wird noch einmal mit Methylenchlorid ($CH_2Cl_2$) extrahiert. Die vereinigten organischen Phasen werden über $Na_2SO_4$ getrocknet, abfiltriert und eingeengt. Das zurückbleibende Öl wird an Kieselgel (Eluent: Cyclohexan/Essigester 50/50) chromatographiert. Man erhält ein leicht gelbes Öl.
Ausbeute: 1,9 g (23 %)
$^1$H-NMR ($CDCl_3$/$TMS_{int}$): δ/ppm = 0,85 (m,15H), 2,48-2,67 (m,1H), 6,8-7,1 (m,4H), 7,22-35 (m,1H), 7,7-8 (m,1H), 8,5 (d,1H), 8,7 (s,1H)

In entsprechender Weise lassen sich die folgenden erfindungsgemäßen Verbindungen der allgemeinen Formel 1

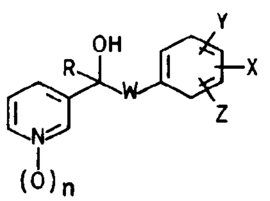

1,

in der n=0 bedeutet, herstellen:

n=0

| Nr. | W | R | 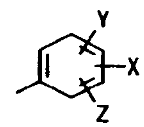 | Physik. Daten |
|---|---|---|---|---|
| 1 | $-CH_2-$ | Cyclopropyl | 2,4-Dichlorphenyl | 98-100°C |
| 2 | $-CH_2-$ | Cyclopropyl | Phenyl | Öl, NMR: 3.25(s,2H) |
| 3 | $-CH_2-$ | Cyclopropyl | 4-Fluorphenyl | Öl, NMR: 3.2(s,2H) |
| 4 | $-CH_2-$ | Cyclopropyl | 4-Chlorphenyl | Öl, NMR: 3,15(s,2H) |
| 5 | $-CH_2-$ | Cyclopropyl | 2-Chlorphenyl | Öl, NMR: 3,25(d,1H),3,5(d,1H) |
| 6 | $-CH_2-$ | Cyclopropyl | 4-Methylphenyl | Öl, NMR: 2,28(s,2H) |
| 7 | $-CH_2CH_2$ | Cyclopropyl | 4-Fluorphenyl | Öl, NMR: 2,0-2,28(m,2H), 2,3-2,48(m,1H), 2,6-2,8(m,1H) |
| 8 | $-CH_2-$ | Cyclopropyl | 2,4-Dimethylphenyl | |
| 9 | $-CH_2-$ | Cyclopropyl | 2,3-Dichlorphenyl | |
| 10 | $-CH_2-$ | Cyclopropyl | 3,4-Dichlorphenyl | |
| 11 | $-CH_2-$ | Cyclopropyl | 2-Chlor-4-fluorphenyl | |
| 12 | $-CH_2-$ | Cyclopropyl | 2-Brom-4-fluorphenyl | |
| 13 | $-CH_2-$ | Cyclopropyl | 4-Cyanophenyl | |
| 14 | $-CH_2-$ | Cyclopropyl | 4-Methoximinophenyl | |
| 15 | $-CH_2-$ | Cyclopropyl | 2-Bromphenyl | |
| 16 | $-CH_2-$ | Cyclopropyl | 2-Chlor-4-methylphenyl | |

| Nr. | W | R | 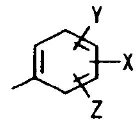 | Physik. Daten |
|---|---|---|---|---|
| 17 | $-CH_2-$ | Cyclopropyl | 2-Trifluormethylphenyl | |
| 18 | $-CH_2-$ | Cyclopropyl | 3-Trifluormethylphenyl | |
| 19 | $-CH_2-$ | Cyclopropyl | 2-Chlor-4-methoxyphenyl | |
| 20 | $-CH_2-$ | Cyclopropyl | 4-Methoxyphenyl | |
| 21 | $-CH_2-$ | Cyclopropyl | 4-tert.-Butylphenyl | |
| 22 | $-CH_2-$ | Cyclopropyl | 2-Fluorphenyl | |
| 23 | $-CH_2-$ | Cyclopropyl | 4-Phenoxyphenyl | |
| 24 | $-CH_2-$ | Cyclopropyl | 2-Fluor-4-chlorphenyl | |
| 25 | $-CH_2-$ | Cyclopropyl | 2,4-Dichlor-3-fluorphenyl | |
| 26 | $-CH_2-$ | Cyclopentyl | 2,4-Dichlorphenyl | öl, NMR: 1.1-2.0(m,9H), 2.7(p,1H), 3,1(d,2H),3.3(d,1H) |
| 27 | $-CH_2-$ | Cyclopentyl | 4-Fluorphenyl | öl, NMR: 1.1-2.0(m,9H), 2.5(p,1H), 3.1(s,2H) |
| 28 | $-CH_2CH_2-$ | Cyclopentyl | 4-Fluorphenyl | öl, NMR: 1.4-2.65(m,14H) |
| 29 | $-CH_2CH_2-$ | Cyclohexyl | 4-Fluorphenyl | öl, NMR: 0.85-2.32(m,15H), 2.48-67(m,1H) |
| 30 | $-CH_2-$ | Cyclohexyl | 2,4-Dichlorphenyl | Fp.: 65°C |
| 31 | $-CH_2-$ | Cyclobutyl | 2,4-Dichlorphenyl | |
| 32 | $-CH_2-$ | 1-Methyl-cyclopropyl | 2,4-Dichlorphenyl | |
| 33 | $-CH_2-$ | 4-Methyl-cyclohexyl | 2,4-Dichlorphenyl | |

| Nr. | W | R | 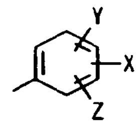 | Physik. Daten |
|---|---|---|---|---|
| 34 | $-CH_2-$ | Cycloheptyl | 4-Fluorphenyl | |
| 35 | $-CH_2-$ | Cyclooctyl | 4-Fluorphenyl | |
| 36 | $-CH_2-$ | Cyclohexyl | 2-Chlor-4-methylphenyl | |
| 37 | $-CH_2-$ | Cyclohexyl | 2,4-Dimethylphenyl | |
| 38 | $-CH_2-$ | Cyclohexyl | 2-Chlorphenyl | Öl, NMR: 0.9-2.27(m,12H), 3,2(d,1H), 3.7(d,1H) |
| 39 | $-CH_2-$ | Cyclohexyl | 2-Bromphenyl | |
| 40 | $-CH_2-$ | Cyclohexyl | 2-Chlor-4-fluorphenyl | |
| 41 | $-CH_2-$ | Cyclohexyl | 2-Brom-4-fluorphenyl | |
| 42 | $-CH_2-$ | Cyclopentyl | 2-Chlor-4-methylphenyl | |
| 43 | $-CH_2-$ | Cyclopentyl | 2,4-Dimethylphenyl | |
| 44 | $-CH_2-$ | Cyclopentyl | 2-Chlorphenyl | Öl: NMR: 1.0-2.0(m,9H), 2.65(p,1H), 3.25(dd,2H) |
| 45 | $-CH_2-$ | Cyclopentyl | 2-Bromphenyl | |
| 46 | $-CH_2-$ | Cyclopentyl | 2-Chlor-4-fluorphenyl | |
| 47 | $-CH_2-$ | Cyclopentyl | 2-Brom-4-fluorphenyl | |
| 48 | $-CH_2-$ | Cyclopentyl | 2-Fluor-4-chlorphenyl | |
| 49 | $-CH_2-$ | Cyclopentyl | 2,3-Dichlorphenyl | |
| 50 | $-CH_2-$ | Cyclohexyl | 2,3-Dichlorphenyl | |
| 51 | $-CH_2CH_2-$ | Cyclopropyl | 4-Chlorphenyl | |
| 52 | $-CH_2CH_2-$ | Cyclopentyl | 4-Chlorphenyl | |

| Nr. | W | R | 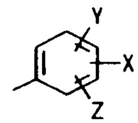 | Physik. Daten |
|---|---|---|---|---|
| 53 | $-CH_2CH_2-$ | Cyclohexyl | 4-Chlorphenyl | |
| 54 | $-CH_2CH_2-$ | Cyclopropyl | 2,4-Dichlorphenyl | |
| 55 | $-CH_2CH_2-$ | Cyclopropyl | Phenyl | |
| 56 | $-CH_2CH_2-$ | Cyclopropyl | 4-Methylphenyl | |
| 57 | $-CH_2CH_2-$ | Cyclopropyl | 2,4-Dimethylphenyl | |
| 58 | $-CH_2CH_2-$ | Cyclopentyl | 2,4-Dichlorphenyl | |
| 59 | $-CH_2CH_2-$ | Cyclohexyl | 4-Methylphenyl | |
| 60 | $-CH_2CH_2-$ | Cyclopentyl | 2,4-Dimethylphenyl | |
| 61 | $-CH_2CH_2-$ | Cyclohex-2-en-1-yl | 4-Fluorphenyl | |
| 62 | $-CH_2CH_2-$ | Cyclohex-2-en-1-yl | 4-Chlorphenyl | |
| 63 | $-CH_2-$ | Cyclopentyl | 4-Chlorphenyl | öl, NMR: 1.15-2.0(m,9H), 2.52(p,1H), 3.12(s,2H) |
| 64 | $-CH_2-$ | Cyclohexyl | 4-Chlorphenyl | öl, NMR: 0.8-2.15(m,12H), 3.12(d,1H), 3.25(d,1H) |
| 65 | $-CH_2-$ | Cyclohexyl | 4-Fluorphenyl | öl, NMR: 3.15(d,1H), 3.25(d,1H) |

Verbindungen der Formel 1, in denen n=1 bedeutet, lassen sich aus den Verbindungen der Tabelle herstellen, indem man diese Verbindungen mit einer Persäure wie meta-Chlorperbenzoesäure oder

Peressigsäure behandelt und anschließend durch eine Wäsche mit wäßriger $NaHCO_3$-Lsg. die entstandene Carbonsäure abtrennt. Dies sei beispielhaft für die Darstellung des N-Oxids der Verbindung 1 der Tabelle durch die folgende Herstellvorschrift veranschaulicht:

Beispiel 3

α-Cyclopropyl-α-(2,4-dichlorbenzyl)-3-pyridylcarbinol-N-Oxid

Zu 2,4 g (0,011 mol) meta-Chlorbenzoesäure in 150 ml Methylenchlorid ($CH_2Cl_2$) werden 3 g (0,01 mol) α-Cyclopropyl-α-(2,4-dichlorbenzyl)-3-pyridylcarbinol (Verbindung Nr. 1) gegeben. Nach Stehenlassen über Nacht bei Raumtemperatur wird von der entstandenen meta-Chlorbenzoesäure abfiltriert, mit $NaHCO_3$-Lösung und $NaHSO_3$-Lösung nachgewaschen und über $Na_2SO_4$ getrocknet. Das nach dem Einengen verbleibende Öl wird an Kieselgel (Eluent: Cyclohexan/Essigester 50/50) chromatographiert. Man erhält leicht braune Kristalle.

Ausbeute: 0,8 g (25 %), Schmelzpunkt: 69 - 73°C

$^1$H-NMR ($CDCl_3$/$TMS_{int}$): δ/ppm = 0,27-60 (m,4H), 1.22-40 (m,1H), 3.15 (d,2H), 3,35 (d,2H), 4,05-60 (s,breit,1H), 7.0-4 (m,5H), 8.0 (d,1H), 8.32 (s,1H)

Die neuen Verbindungen zeichnen sich, allgemein ausgedrückt, durch eine hervorragende Wirksamkeit gegen ein breites Spektrum von pflanzenpathogenen Pilzen, insbesondere aus der Klasse der Ascomyceten und Basidiomyceten, aus. Sie sind zum Teil systemisch wirksam und können als Blatt- und Bodenfungizide eingesetzt werden.

Besonders interessant sind die fungiziden Verbindungen für die Bekämpfung einer Vielzahl von Pilzen an verschiedenen Kulturpflanzen oder ihren Samen, insbesondere Weizen, Roggen, Gerste, Hafer, Reis, Mais, Rasen, Baumwolle, Soja, Kaffee, Zuckerrohr, Obst und Zierpflanzen im Gartenbau, Weinbau sowie Gemüse - wie Gurken, Bohnen und Kürbisgewächse -.

Die neuen Verbindungen sind insbesondere geeignet zur Bekämpfung folgender Pflanzenkrankheiten:

Erysiphe graminis (echter Mehltau) in Getreide,
Erysiphe cichoracearum und Sphaerotheca fuliginea an Kürbisgewächsen,
Podosphaera leucotricha an Äpfeln,
Uncinula necator an Reben,
Puccinia-Arten an Getreide,
Rhizoctonia-Arten an Baumwolle und Rasen,
Ustilago-Arten an Getreide und Zuckerrohr,
Venturia inaequalis (Schorf) an Äpfeln,
Helminthosporium-Arten an Getreide,
Septoria nodorum an Weizen,
Botrytis cinerea (Grauschimmel) an Erdbeeren, Reben,
Cercospora arachidicola an Erdnüssen,
Pseudocercosporella herpotrichoides an Weizen, Gerste,
Pyricularia oryzae an Reis,
Phytophthora infestans an Kartoffeln und Tomaten,
Fusarium- und Verticillium-Arten an verschiedenen Pflanzen,
Plasmopara viticola an Reben,
Alternaria-Arten an Gemüse und Obst.

Die Verbindungen werden angewendet, indem man die Pflanzen mit den Wirkstoffen besprüht oder bestäubt oder die Samen der Pflanzen mit den Wirkstoffen behandelt. Die Anwendung erfolgt vor oder nach der Infektion der Pflanzen oder Samen durch die Pilze. Es werden die Pilze oder die vor Pilzbefall zu schützenden Pflanzen, Saatgüter, Materialien oder der Erdboden mit einer fungizid wirksamen Menge des Wirkstoffs behandelt.

Die neuen Substanzen können in die üblichen Formulierungen übergeführt werden, wie Lösungen, Emulsionen, Suspensionen, Stäube, Pulver, Pasten und Granulate. Die Anwendungsformen richten sich ganz nach den Verwendungszwecken; sie sollen in jedem Fall eine feine und gleichmäßige Verteilung der wirksamen Substanz gewährleisten. Die Formulierungen werden in bekannter Weise hergestellt, z.B. durch Verstrecken des Wirkstoffs mit Lösungsmitteln und/oder Trägerstoffen, gegebenenfalls unter Verwendung von Emulgiermitteln und Dispergiermitteln, wobei im Falle der Benutzung von Wasser als Verdünnungsmittel auch andere organische Lösungsmittel als Hilfslösungsmittel verwendet werden können. Als Hilfsstoffe kommen dafür im wesentlichen in Frage: Lösungsmittel wie Aromaten (z.B. Xylol), chlorierte Aromaten (z.B. Chlorbenzole), Paraffine (z.B. Erdölfraktionen), Alkohole (z.B. Methanol, Butanol), Ketone (z.B. Cyclohexanon), Amine (z.B. Ethanolamin, Dimethylformamid) und Wasser; Trägerstoffe wie natürliche Gesteinsmehle (z.B. Kaoline, Tonerden, Talkum, Kreide) und synthetische Gesteinsmehle (z.B. hochdisperse Kieselsäure, Silikate); Emulgiermittel, wie nichtionogene und anionische Emulgatoren (z.B. Polyoxyethylen-Fettalkohol-Ether, Alkylsulfonate und Arylsulfonate) und Dispergiermittel, wie Lignin, Sulfitablaugen und Methylcellulose.

Die fungiziden Mittel enthalten im allgemeinen zwischen 0,1 und 95, vorzugsweise zwischen 0,5 und 90 Gew.% Wirkstoff.

Die Aufwandmengen liegen je nach Art des gewünschten Effektes zwischen 0,02 und 3 kg Wirkstoff oder mehr je ha. Die neuen Verbindungen können auch im Materialschutz (Holzschutz) eingesetzt werden, z.B. gegen Paecilomyces variotii. Bei der Saatgutbehandlung werden im allgemeinen Wirkstoffmengen von 0,001 bis 50 g, vorzugsweise 0,01 bis 10 g, je Kilogramm Saatgut benötigt.

Die Mittel bzw. die daraus hergestellten gebrauchsfertigen Zubereitungen, wie Lösungen, Emulsionen, Suspensionen, Pulver, Stäube, Pasten oder Granulate werden in bekannter Weise angewendet, beispielsweiise durch Versprühen, Vernebeln, Verstäuben, Verstreuen, Beizen oder Gießen.

Beispiele für solche Zubereitungen sind:

I. Man vermischt 90 Gew.-Teile der Verbindung Nr. 1 mit 10 Gew.-Teilen N-Methyl-α-pyrrolidon und erhält eine Lösung, die zur Anwendung in Form kleinster Tropfen geeignet ist.

II. 20 Gew.-Teile der Verbindung Nr. 5 werden in einer Mischung gelöst, die aus 80 Gew.-Teilen Xylol, 10 Gew.-Teilen des Anlagerungsproduktes von 8 bis 10 Mol Ethylenoxid an 1 Mol Ölsäure-N-monoethanolamid, 5 Gew.-Teilen Calciumsalz der Dodecylbenzolsulfonsäure und 5 Gew.-Teilen des Anlagerungsproduktes und 40 Mol Ethylenoxid an 1 Mol Ricinusöl besteht. Durch Ausgießen und feines Verteilen der Lösung in Wasser erhält man eine wäßrige Dispersion.

III. 20 Gew.-Teile der Verbindung Nr. 1 werden in einer Mischung gelöst, die aus 40 Gew.-Teilen Cyclohexanon, 30 Gew.-Teilen Isobutanol, 20 Gew.-Teilen des Anlagerungsproduktes von 40 Mol Ethylenoxid an 1 Mol Ricinusöl besteht. Durch Eingießen und feines Verteilen der Lösung in Wasser erhält man eine wäßrige Dispersion.

IV. 20 Gew.-Teile der Verbindung Nr. 5 werden in einer Mischung gelöst, die aus 25 Gew.-Teilen Cyclohexanol, 65 Gew.-Teilen einer Mineralölfraktion vom Siedepunkt 210 bis 280°C und 10 Gew.-Teilen des Anlagerungsproduktes von 40 Mol Ethylenoxid an 1 Mol Ricinusöl besteht. Durch Eingießen und feines Verteilen der Lösung in Wasser erhält man eine wäßrige Dispersion.

V. 80 Gew.-Teile der Verbindung Nr. 1 werden mit 3 Gew.-Teilen des Natriumsalzes der Diisobutylnaphthalin-α-sulfonsäure, 10 Gew.-Teilen des Natriumsalzes einer Ligninsulfonsäure aus einer Sulfitablauge und 7 Gew.-Teilen pulverförmigem Kieselsäuregel gut vermischt und in einer Hammermühle vermahlen. Durch feines Verteilen der Mischung in Wasser erhält man eine Spritzbrühe.

VI. 3 Gew.-Teile der Verbindung Nr. 5 werden mit 97 Gew.-Teilen feinteiligem Kaolin innig vermischt. Man erhält auf diese Weise ein Stäubemittel, das 3 Gew.% des Wirkstoffs enthält.

VII. 30 Gew.-Teile der Verbindung Nr. 1 werden mit einer Mischung aus 92 Gew.-Teilen pulverförmigem Kieselsäuregel und 8 Gew.-Teilen Paraffinöl, das auf die Oberfläche dieses Kieselsäuregels gesprüht wurde, innig vermischt. Man erhält auf diese Weise eine Aufbereitung des Wirkstoffs mit guter Haftfähigkeit.

VIII. 40 Gew.-Teile der Verbindung Nr. 5 werden mit 10 Gew.-Teilen Natriumsalz eines Phenolsulfonsäure-harnstoff-formaldehyd-Kondensates, 2 Gew.-Teilen Kieselgel und 48 Gew.-Teilen Wasser innig vermischt. Man erhält eine stabile wäßrige Dispersion. Durch Verdünnen mit Wasser erhält man eine wäßrige Dispersion.

IX. 20 Gew.-Teile der Verbindung Nr. 1 werden mit 2 Gew.-Teilen Calciumsalz der Dodecylbenzolsulfonsäure, 8 Gew.-Teilen Fettalkoholpolyglykolether, 2 Gew.-Teilen Natriumsalz eines Phenolsulfonsäure-harnstoff-formaldehyd-Kondensats und 68 Gew.-Teilen eines paraffinischen Mineralöls innig vermischt. Man erhält eine stabile ölige Dispersion.

Die erfindungsgemäßen Mittel können in diesen Anwendungsformen auch zusammen mit anderen Wirkstoffen vorliegen, wie z.B. Herbiziden, Insektiziden, Wachstumsregulatoren und Fungiziden, oder auch mit Düngemitteln vermischt und ausgebracht werden. Beim Vermischen mit Fungiziden erhält man dabei in vielen Fällen eine Vergrößerung des fungiziden Wirkungsspektrums.

Anwendungsbeispiele

Als Vergleichswirkstoff wurde α-(4-Chlorphenyl)-α-cyclopropyl-3-pyridinmethanol - bekannt aus US 3 396 224 - benutzt.

Anwendungsbeispiel 1

Wirksamkeit gegen Plasmopara viticola

Blätter von Topfreben der Sorte "Müller Thurgau" wurden mit wäßriger Spritzbrühe, die 80 % Wirkstoff und 20 % Emulgiermittel in der Trockensubstanz enthielt, besprüht. Um die Wirkungsdauer der Wirkstoffe beurteilen zu können, wurden die Pflanzen nach dem Antrocknen des Spritzbelages 8 Tage im Gewächshaus aufgestellt. Erst dann wurden die Blätter mit einer Zoosporenaufschwemmung von Plasmopara viticola (Rebenperonospora) infiziert. Danach wurden die Reben zunächst für 48 Stunden in einer wasserdampfgesättigten Kammer bei 24°C und anschließend für 5 Tage in einem Gewächshaus mit Temperaturen zwischen 20 und 30°C aufgestellt. Nach dieser Zeit wurden die Pflanzen zur Beschleunigung des Sporangienträgerausbruches abermals für 16 Stunden in der feuchten Kammer aufgestellt. Dann erfolgte die Beurteilung des Ausmaßes der Pilzentwicklung auf den Blattunterseiten.

Das Ergebnis zeigt, daß die Wirkstoffe 1 und 5 bei der Anwendung als 0,025 %ige (Gew.%) Spritzbrühe eine bessere fungizide Wirkung zeigen (90 %) als der bekannte Vergleichswirkstoff A (60 %).

Anwendungsbeispiel 2

Wirksamkeit gegen Botrytis cinerea an Paprika

Paprikasämlinge der Sorte "Neusiedler Ideal Elite" wurden, nachdem sich 4-5 Blätter gut entwickelt hatten, mit wäßrigen Suspensionen, die 80 % Wirkstoff und 20 % Emulgiermittel in der Trockensubstanz enthielten, tropfnaß gespritzt. Nach 8-tägiger Kultur der behandelten Pflanzen im Gewächshaus wurden sie mit einer Sporensuspension von Botrytis cinerea, die $1{,}7 \times 10^6$ Sporen/ml in einer 2 %igen Biomalzlösung enthielt, inoculiert. Anschließend wurden sie in eine Klimakammer mit 22-24°C und hoher Luftfeuchtigkeit gestellt. Nach 5 Tagen konnte das Ausmaß der Pilzentwicklung ausgewertet werden. (Pilzbefall der Blattflächen).

Das Ergebnis zeigt, daß der Wirkstoff 1 bei der Anwendung als 0,025 %ige Spritzbrühe eine bessere fungizide Wirkung zeigt (100 %) als der bekannte Vergleichswirkstoff A (75 %).

## Patentansprüche

1. Verbindungen der Formel 1

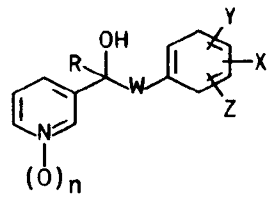

1,

in der R
für gegebenenfalls $C_1$-$C_4$-alkylsubstituiertes $C_3$-$C_8$-Cycloalkyl oder für gegebenenfalls $C_1$-$C_4$-alkylsubstituiertes $C_3$-$C_8$-Cycloalkenyl steht,
X, Y, Z
unabhängig voneinander Wasserstoff, Halogen, $C_1$-$C_4$-Alkyl, $C_1$-$C_4$-Alkoxy, $C_1$-$C_4$-Alkoximino, Halogen-$C_1$-$C_4$-alkyl, Cyano, Nitro oder gegebenenfalls durch Halogen substituiertes Phenyl oder gegebenenfalls durch Halogen substituiertes Phenoxy bedeuten,
W
für eine der Gruppen $-CH_2-$ oder $-CH_2CH_2-$ steht und
n
0 oder 1 bedeutet,
sowie deren pflanzenverträgliche Säureadditionssalze.

2. Verfahren zur Herstellung eines Pyridins der Formel 1

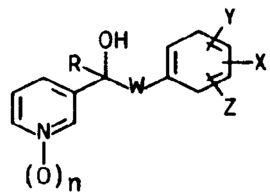

1,

in der R
für gegebenenfalls $C_1$-$C_4$-alkylsubstituiertes $C_3$-$C_8$-Cycloalkyl oder für gegebenenfalls $C_1$-$C_4$-alkylsubstituiertes $C_3$-$C_8$-Cycloalkenyl steht,
X, Y, Z
unabhängig voneinander Wasserstoff, Halogen, $C_1$-$C_4$-Alkyl, $C_1$-$C_4$-Alkoxy, $C_1$-$C_4$-Alkoximino, Halogen-$C_1$-$C_4$-alkyl, Cyano, Nitro oder gegebenenfalls durch Halogen substituiertes Phenyl oder gegebenenfalls durch Halogen substituiertes Phenoxy bedeuten,
W
für eine der Gruppen $-CH_2-$ oder $-CH_2CH_2-$ steht und
n
0 oder 1 bedeutet,
dadurch gekennzeichnet, daß man
a) ein Acylpyridin der Formel 2

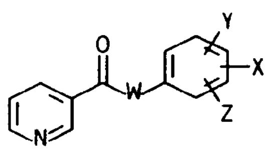

2,

in der X,Y,Z
unabhängig voneinander Halogen, $C_1$-$C_4$-Alkyl, $C_1$-$C_4$-Alkoxy, $C_1$-$C_4$-Alkoxy, $C_1$-$C_4$-Alkoximino, Halogen-$C_1$-$C_4$-alkyl, Cyano, Nitro oder gegebenenfalls durch Halogen substituiertes Phenyl oder gegebenenfalls durch Halogen substituiertes Phenoxy bedeuten können und
W
für eine der Gruppen $-CH_2-$ oder $-CH_2CH_2-$ steht, in Gegenwart eines Lösungsmittels mit einer metallorganischen Verbindung der Formel 3

M-R 3,

in der M = Li, Na, K, MgCl oder MgBr,
und R
gegebenenfalls $C_1$-$C_4$-alkylsubstituiertes $C_3$-$C_8$-Cycloalkyl oder für gegebenenfalls $C_1$-$C_4$-alkylsubstituiertes $C_3$-$C_8$-Cycloalkenyl bedeutet,
umsetzt oder
b) ein Acylpyridin der Formel 4

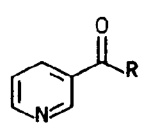

4,

in der R
gegebenenfalls $C_1$-$C_4$-alkylsubstituiertes $C_3$-$C_8$-Cycloalkyl oder gegebenenfalls $C_1$-$C_4$-alkylsubstituiertes $C_3$-$C_8$-Cycloalkenyl bedeutet, in Gegenwart eines Lösungsmittels mit einer metallorganischen Verbindung der Formel 5 umsetzt

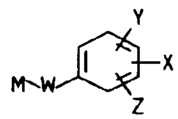

5,

in der M = Li, Na, K, MgCl oder MgBr,

X,Y,Z

unabhängig voneinander Halogen, $C_1$-$C_4$-Alkyl, $C_1$-$C_4$-Alkoxy, $C_1$-$C_4$-Alkoximino, Halogen-$C_1$-$C_4$-alkyl, Cyano, Nitro oder gegebenenfalls durch Halogen substituiertes Phenyl oder gegebenenfalls durch Halogen substituiertes Phenoxy bedeuten und

W

für eine der Gruppen $-CH_2-$ oder $-CH_2CH_2-$ steht und die gemäß a) oder b) erhaltene Verbindung der Formel 1, für den Fall, daß n die Zahl 1 bedeutet,

mit Persäure oder einem Wasserstoffperoxid/Säure-Gemisch behandelt.

3. Fungizid, enthaltend einen festen oder flüssigen Trägerstoff und eine fungizid wirksame Menge einer Verbindung der Formel 1

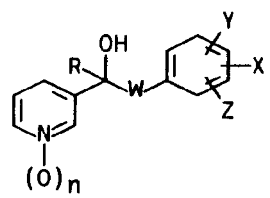

1,

in der R

für gegebenenfalls $C_1$-$C_4$-alkylsubstituiertes $C_3$-$C_8$-Cycloalkyl oder für gegebenenfalls $C_1$-$C_4$-alkylsubstituiertes $C_3$-$C_8$-Cycloalkenyl steht,

X, Y, Z

unabhängig voneinander Wasserstoff, Halogen, $C_1$-$C_4$-Alkyl, $C_1$-$C_4$-Alkoxy, $C_1$-$C_4$-Alkoximino, Halogen-$C_1$-$C_4$-alkyl, Cyano, Nitro oder gegebenenfalls durch Halogen substituiertes Phenyl oder gegebenenfalls durch Halogen substituiertes Phenoxy bedeuten,

W

eine der Gruppen $-CH_2-$ oder $-CH_2CH_2-$ und

n

0 oder 1 bedeutet,

oder dessen pflanzenverträgliches Säureadditionssalz.

4. Verfahren zur Bekämpfung von Pilzen, dadurch gekennzeichnet, daß man eine fungizid wirksame Menge einer Verbindung der Formel 1

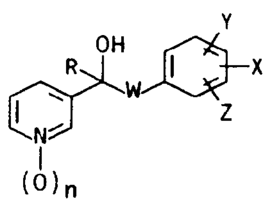

1,

in der R

für gegebenenfalls $C_1$-$C_4$-alkylsubstituiertes $C_3$-$C_8$-Cycloalkyl oder für gegebenenfalls $C_1$-$C_4$-alkylsubstituiertes $C_3$-$C_8$-Cycloalkenyl steht,

X, Y, Z

unabhängig voneinander Wasserstoff, Halogen, $C_1$-$C_4$-Alkyl, $C_1$-$C_4$-Alkoxy, $C_1$-$C_4$-Alkoximino, Halogen-$C_1$-$C_4$-alkyl, Cyano, Nitro oder gegebenenfalls durch Halogen substituiertes Phenyl oder gegebenenfalls durch Halogen substituiertes Phenoxy bedeuten,

W
eine der Gruppen $-CH_2-$ oder $-CH_2CH_2-$ und
n
0 oder 1 bedeutet, oder dessen pflanzenverträgliches Säureadditionssalz
auf die Pilze oder auf durch Pilzbefall bedrohte Materialien, Pflanzen, Saatgüter oder den Erdboden einwirken läßt.

**5.** Verbindung der Formel 1 gemäß Anspruch 1, dadurch gekennzeichnet, daß R Cyclopropyl, X, Y, Z 2,4-Dichlor, W $-CH_2-$ und n 0 bedeutet.

**6.** Verbindung der Formel 1 gemäß Anspruch 1, dadurch gekennzeichnet, daß R Cyclopropyl, X, Y, Z 2-Chlor, W $-CH_2-$ und n 0 bedeutet.

## Claims

**1.** A compound of the formula I

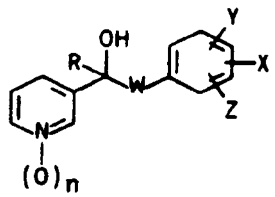

1,

where
R is unsubstituted or $C_1$-$C_4$-alkyl-substituted $C_3$-$C_8$-cycloalkyl, or unsubstituted or $C_1$-$C_4$-alkyl-substituted $C_3$-$C_8$-cycloalkenyl,
X, Y and Z, independently of one another, are hydrogen, halogen, $C_1$-$C_4$-alkyl, $C_1$-$C_4$-alkoxy, $C_1$-$C_4$-alkoximino, halo-$C_1$-$C_4$-alkyl, cyano, nitro, unsubstituted or halogen-substituted phenyl, or unsubstituted or halogen-substituted phenoxy,
W is $-CH_2-$ or $-CH_2CH_2-$, and
n is 0 or 1,
or a plant-tolerated acid-addition salt thereof.

**2.** A process for the preparation of a pyridine of the formula I

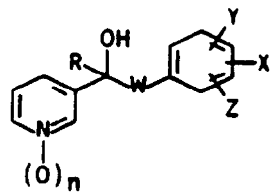

1,

where
R is unsubstituted or $C_1$-$C_4$-alkyl-substituted $C_3$-$C_8$-cycloalkyl, or unsubstituted or $C_1$-$C_4$-alkyl- substituted $C_3$-$C_8$-cycloalkenyl,
X, Y and Z, independently of one another, are hydrogen, halogen, $C_1$-$C_4$-alkyl, $C_1$-$C_4$-alkoxy, $C_1$-$C_4$-alkoximino, halo-$C_1$-$C_4$-alkyl, cyano, nitro, unsubstituted or halogen-substituted phenyl, or unsubstituted or halogen-substituted phenoxy,
W is $-CH_2-$ or $-CH_2CH_2-$, and
n is 0 or 1,
which comprises
a) reacting an acylpyridine of the formula 2

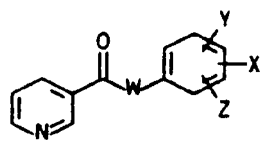

2,

where X, Y and Z, independently of one another, are halogen, $C_1$-$C_4$-alkyl, $C_1$-$C_4$-alkoxy, $C_1$-$C_4$-alkoximino, halo-$C_1$-$C_4$-alkyl, cyano, nitro, unsubstituted or halogen-substituted phenyl or unsubstituted or halogen-substituted phenoxy, and
W is $-CH_2-$ or $-CH_2CH_2-$,
with an organometallic compound of the formula 3

M-R 3,

where M is Li, Na, K, MgCl or MgBr,
and R is unsubstituted or $C_1$-$C_4$-alkyl-substituted $C_3$-$C_8$-cycloalkyl or unsubstituted or $C_1$-$C_4$-alkyl-substituted $C_3$-$C_8$-cycloalkenyl,
in the presence of a solvent, or
b) reacting an acylpyridine of the formula 4

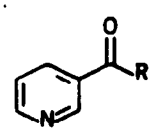

4,

where
R is unsubstituted or $C_1$-$C_4$-alkyl-substituted $C_3$-$C_8$-cycloalkyl or unsubstituted or $C_1$-$C_4$-alkyl-substituted $C_3$-$C_8$-cycloalkenyl,
with an organometallic compound of the formula 5

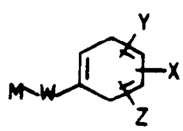

5,

where M is Li, Na, K, MgCl or MgBr,
X, Y and Z, independently of one another, are halogen, $C_1$-$C_4$-alkyl, $C_1$-$C_4$-alkoxy, $C_1$-$C_4$-alkoximino, halo-$C_1$-$C_4$-alkyl, cyano, nitro, unsubstituted or halogen-substituted phenyl or unsubstituted or halogen-substituted phenoxy and
W is $-CH_2-$ or $-CH_2CH_2-$, in the presence of a solvent,
and treating the compound of the formula 1 obtained from a) or b), in the case where n is 1, with a peracid or a hydrogen peroxide/acid mixture.

3. A fungicide containing a solid or liquid carrier and a fungicidally effective amount of a compound of the formula 1

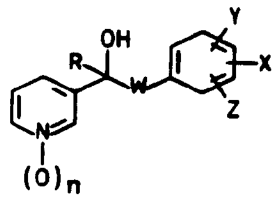

1

where
R is unsubstituted or $C_1$-$C_4$-alkyl-substituted $C_3$-$C_8$-cycloalkyl, or unsubstituted or $C_1$-$C_4$-alkyl-substituted $C_3$-$C_8$-cycloalkenyl,
X, Y and Z, independently of one another, are hydrogen, halogen, $C_1$-$C_4$-alkyl, $C_1$-$C_4$-alkoxy, $C_1$-$C_4$-al-

koximino, halo-$C_1$-$C_4$-alkyl, cyano, nitro, unsubstituted or halogen-substituted phenyl, or unsubstituted or halogen-substituted phenoxy,
W is $-CH_2-$ or $-CH_2CH_2-$, and
n is 0 or 1,
or a plant-tolerated acid-addition salt thereof.

4. A method for controlling fungi, which comprises allowing a fungicidally effective amount of a compound of the formula I

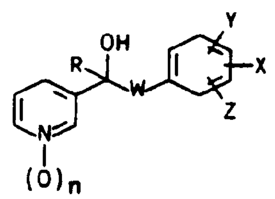

1

where
R is unsubstituted or $C_1$-$C_4$-alkyl-substituted $C_3$-$C_8$-cycloalkyl, or unsubstituted or $C_1$-$C_4$-alkyl-substituted $C_3$-$C_8$-cycloalkenyl,
X, Y and Z, independently of one another, are hydrogen, halogen, $C_1$-$C_4$-alkyl, $C_1$-$C_4$-alkoxy, $C_1$-$C_4$-alkoximino, halo-$C_1$-$C_4$-alkyl, cyano, nitro, unsubstituted or halogen-substituted phenyl, or unsubstituted or halogen-substituted phenoxy,
W is $-CH_2-$ or $-CH_2CH_2-$, and
n is 0 or 1,
or a plant-tolerated acid-addition salt thereof,
to act on the fungi or on the materials, plants, seed or soil threatened by fungal attack.

5. A compound of the formula 1 as claimed in claim 1, wherein R is cyclopropyl, X, Y and Z are 2,4-dichloro, W is $-CH_2-$ and n is 0.

6. A compound of the formula 1 as claimed in claim 1, wherein R is cyclopropyl, X, Y and Z are 2-chloro, W is $-CH_2-$ and n is 0.

**Revendications**

1. Composés de formule I

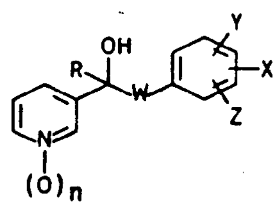

I,

dans laquelle R représente un groupe cycloalkyle en C3-C8 éventuellement substitué par un groupe alkyle en C1-C4 ou un groupe cycloalcényle en C3-C8 éventuellement substitué par un groupe alkyle en C1-C4,
X, Y, Z représentent chacun, indépendamment les uns des autres, l'hydrogène, un halogène, un groupe alkyle en C1-C4, alcoxy en C1-C4, alcoximino en C1-C4, halogénoalkyle en C1-C4, cyano, nitro ou un groupe phényle éventuellement substitué par des halogènes ou un groupe phénoxy éventuellement substitué par des halogènes,
W représente l'un des groupes $-CH_2-$ ou $-CH_2CH_2-$, et
n est égal à 0 ou 1,
et leurs sels formés par addition avec des acides tolérés par les végétaux.

2. Procédé de préparation d'une pyridine de formule I

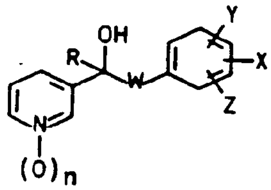

1,

dans laquelle R représente un groupe cycloalkyle en C3-C8 éventuellement substitué par un groupe alkyle en C1-C4 ou un groupe cycloalcényle en C3-C8 éventuellement substitué par un groupe alkyle en C1-C4,
X, Y, Z représentent chacun, indépendamment les uns des autres, l'hydrogène, un halogène, un groupe alkyle en C1-C4, alcoxy en C1-C4, alcoximino en C1-C4, halogénoalkyle en C1-C4, cyano, nitro ou un groupe phényle éventuellement substitué par des halogènes ou un groupe phénoxy éventuellement substitué par des halogènes,
W représente l'un des groupes $-CH_2-$ ou $-CH_2CH_2-$, et
n est égal à 0 ou 1 ,
caractérisé en ce que:

a) on fait réagir une acylpyridine de formule 2

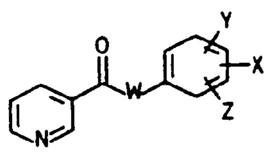

2,

dans laquelle X, Y, Z représentent chacun, indépendamment les uns des autres, un halogène, un groupe alkyle en C1-C4, alcoxy en C1-C4, alcoximino en C1-C4, halogénoalkyle en C1-C4, cyano, nitro ou un groupe phényle éventuellement substitué par des halogènes ou un groupe phénoxy éventuellement substitué par des halogènes, et
W représente l'un des groupes $-CH_2-$ ou $-CH_2CH_2-$, en présence d'un solvant, avec un dérivé organométallique de formule 3

M-R 3,

dans laquelle M = Li, Na, K, MgCl ou MgBr,
et R représente un groupe cycloalkyle en C3-C8 éventuellement substitué par un groupe alkyle en C1-C4 ou un groupe cycloalcényle en C3-C8 éventuellement substitué par un groupe alkyle en C1-C4, ou bien

b) on fait réagir une acylpyridine de formule 4

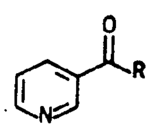

4,

dans laquelle R représente un groupe cycloalkyle en C3-C8 éventuellement substitué par un groupe alkyle en C1-C4 ou un groupe cycloalcényle en C3-C8 éventuellement substitué par un groupe alkyle en C1-C4, en présence d'un solvant, avec un dérivé organo-métallique d formule 5

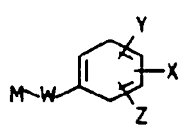

5,

dans laquelle M = Li, Na, K, MgCl ou MgBr,

X, Y, Z représentent chacun, indépendamment les uns des autres, un halogène un groupe alkyle en C1-C4, alcoxy en C1-C4, alcoximino en C1-C4, halogénoalkyle en C1-C4, cyano, nitro ou un groupe phényle éventuellement substitué par des halogènes ou un groupe phénoxy éventuellement substitué par des halogènes, et
W représente l'un des groupes $-CH_2-$ ou $-CH_2CH_2-$,
et dans les cas où n = 1, on traite le composé de formule 1 obtenu selon a) ou b), par un peracide ou par un mélange peroxyde d'hydrogène/acide.

3. Produit fongicide contenant un véhicule solide ou liquide et une quantité fongicide efficace d'un composé de formule 1

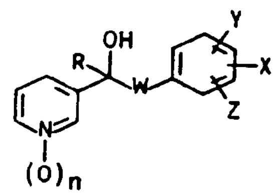

1,

dans laquelle R représente un groupe cycloalkyle en C3-C8 éventuellement substitué par un groupe alkyle en C1-C4 ou un groupe cycloalcényle en C3-C8 éventuellement substitué par un groupe alkyle en C1-C4,
X, Y, Z représentent chacun, indépendamment les uns des autres, un halogène, un groupe alkyle en C1-C4, alcoxy en C1-C4, alcoximino en C1-C4, halogénoalkyle en C1-C4, cyano, nitro ou un groupe phényle éventuellement substitué par des halogènes ou un groupe phénoxy éventuellement substitué par des halogènes,
W représente l'un des groupes -CH- ou $-CH_2CH_2-$, et
n est égal à 0 ou 1,
ou d'un sel d'un tel composé formé par addition avec un acide toléré par les végétaux.

4. Procédé pour combattre les mycètes, caractérisé en ce que l'on fait agir une quantité fongicide efficace d'un composé de formule I

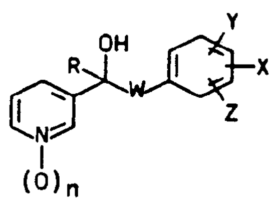

1,

dans laquelle R représente un groupe cycloalkyle en C3-C8 éventuellement substitué par un groupe alkyle en C1-C4 ou un groupe cycloalcényle en C3-C8 éventuellement substitué par un groupe alkyle en C1-C4,
X, Y, Z représentent chacun, indépendamment les uns des autres, l'hydrogène, un halogène, un groupe alkyle en C1-C4, alcoxy en C1-C4, alcoximino en C1-C4, halogénoalkyle en C1-C4, cyano, nitro ou un groupe phényle éventuellement substitué par des halogènes ou un groupe phénoxy éventuellement substitué par des halogènes,
W représente l'un des groupes $-CH_2-$ ou $-CH_2CH_2-$, et
n est égal à 0 ou 1, ou de l'un de ses sels formé par addition avec un acide toléré par les végétaux, sur les mycètes ou sur les matières, plantes, semences ou sol menacés par une attaque par les mycètes.

5. Composé de formule I de la revendication 1, caractérisé en ce que
R représente un groupe cyclopropyle,
X, Y, Z représentent un substituant 2,4-dichloro,
W représente $-CH_2-$ et n est égal à 0.

6. Composé de formule I de la revendication 1, caractérisé en ce que
R représente un groupe cyclopropyle,
X, Y, Z représentent un substituant 2-chloro,
W représente-$CH_2-$, et
n est égal à 0.